# EUROPEAN PATENT APPLICATION

(11) **EP 1 876 264 A1**
(43) Date of publication of application: **09.01.2008**
(21) Application number: 06731447.6
(22) Date of filing: 07.04.2006
(51) Int. Cl.: C25B 3/10

(54) **PROCESS FOR PRODUCING COMPOUND HAVING FLUOROSULFONYL GROUP THROUGH COUPLING REACTION**

(30) Priority: 18.04.2005 JP 2005119721
(71) Applicant: Asahi Glass Company, Limited, Chiyoda-ku, Tokyo 100-8405 (JP)
(72) Inventor: IWAYA, Masao c/o Asahi Glass Company, Limited, Yokohama-shi, Kanagawa 2218755 (JP); OKAMOTO, Hidekazu c/o Asahi Glass Company, Limited, Yokohama-shi, Kanagawa 2218755 (JP); OHARU, Kazuya, c/o Asahi Glass Company, Limited, Yokohama-shi, Kanagawa 2218755 (JP)
(74) Representative: Müller-Boré & Partner Patentanwälte
(86) International application number: PCT/JP2006/307500
(87) International publication number: WO 2006/115018

(57) **Abstract**

To provide a method for producing a fluorinated compound having a fluorosulfonyl group by coupling reaction.

A method for producing a compound represented by the formula [(FSO₂-)ₐ(Z-)₃₋ₐC-]ₙR(-COY)_{b-n}, which comprises subjecting a compound represented by the formula (FSO₂-)ₐ(Z-)₃₋ₐC(-COX) and a compound represented by the formula R(-COY)_{b} to photocoupling reaction when X and Y are a fluorine atom or to electrolytic coupling reaction when X and Y are each independently a hydroxyl group or a group represented by the formula -OM, provided that X and Y are a fluorine atom, a hydroxyl group or a group represented by the formula -OM, Z is a hydrogen atom, a fluorine atom, a chlorine atom or a monovalent organic group, R is a b-valent organic group, M is an alkali metal atom, "a" is an integer of from 1 to 3, and "b" and "n" are each independently an integer of from 1 to 4, provided that b≧n.

## Description

### TECHNICAL FIELD

The present invention relates to a method for producing a compound having a fluorosulfonyl group by coupling reaction.

### BACKGROUND ART

A fluorinated compound having a fluorosulfonyl group (hereinafter sometimes referred to simply as -SO₂F) is useful as a functional material such as a surfactant or a monomer. In a method for producing a fluorinated compound having -SO₂F, a cyclic compound such as perfluoroethane sultone obtained by reacting a fluorinated olefin such as tetrafluoroethylene with SO₃ is used in some cases.

For example, FSO₂CF₂CF₂OCF=CF₂ which is a monomer for a fluorosulfonic acid polymer for an ion exchange membrane is produced by using FSO₂CF₂CF₂OCF(CF₃)COF obtained by reacting perfluoroethane sultone with hexafluoropropylene oxide.

Further, a method has been reported of subjecting FSO₂CF₂CF₂OCF(CF₃)COOH and CF₃CF₂CF₂COOH to heterocoupling by electrolytic coupling reaction to obtain FSO₂CF₂CF₂OCF(CF₃)CF₂CF₂CF₃ (Non-Patent Document 1).

Non-Patent Document 1: V.F. Cherstkov et al, Izvestiya Akademii Nauk SSSR, Seriya, Khimicheskaya, 1990, 10, 2448-2449.

### DISCLOSURE OF THE INVENTION

### PROBLEMS TO BE SOLVED BY THE INVENTION

A fluorinated carboxylic acid having -SO₂F disclosed in the above document is only a compound having -SO₂F at the δ-position of the carboxyl group such as FSO₂CF₂CF₂OCF(CF₃)COOH. The above document failed to disclose fluorinated carboxylic acid analogues having -SO₂F at the α-position such as FSO₂CF₂COOH having higher activity than the above compound. Therefore, an attempt to subject such a fluorinated carboxylic acid analogue and another carboxylic acid analogue to heterocoupling by electrolytic coupling reaction has been totally unknown including its success and failure.

### MEANS TO SOLVE THE PROBLEMS

The present inventors have found that a fluorinated carboxylic acid analogue having -SO₂F at the α-position and another carboxylic acid analogue can be subjected to heterocoupling by photocoupling reaction or electrolytic coupling reaction.

Namely, the present invention provides the following:
(1) A method for producing a compound represented by the following formula (3), which comprises subjecting a compound represented by the following formula (1) and a compound represented by the following formula (2) to photocoupling reaction when X and Y are a fluorine atom, or to electrolytic coupling reaction when X and Y are each independently a hydroxyl group or a group represented by the formula -OM:

   (FS0₂-)ₐ(Z-)₃₋ₐC(-COX) (1),

   R(-COY)_{b} (2),

   [(FSO₂-)ₐ(Z-)₃₋ₐC-]ₙR(-COY)_{b-n} (3)

   wherein symbols are as follows:
   X: a fluorine atom, a hydroxyl group or a group represented by the formula -OM;
   Y: a fluorine atom, a hydroxyl group or a group represented by the formula -OM;
   Z: a hydrogen atom, a fluorine atom, a chlorine atom or a monovalent organic group;
   R: a b-valent organic group;
   M: an alkali metal atom;
   a: an integer of from 1 to 3, and
   b and n: each independently an integer of from 1 to 4, provided that b≧n.
(2) The production method according to the above (1), wherein R is a group selected from the group consisting of a fluoroaliphatic hydrocarbon group, a fluoro(partially chloroaliphatic hydrocarbon) group, a fluoro(etheric oxygen atom-containing aliphatic hydrocarbon) group and a fluoro(partially chloro(etheric oxygen atom-containing aliphatic hydrocarbon)) group, and is a C₁₋₁₀ b-valent organic group.
(3) The production method according to the above (1) or (2), wherein the compound represented by the formula (2) is used in an amount of from (0.01/b to 1.0/b) mol (wherein b is as defined above) per 1 mol of the compound represented by the formula (1).
(4) A method for producing a compound represented by the following formula (3-1), which comprises subjecting a compound represented by the following formula (1-1) and a compound represented by the following formula (2-1) to electrolytic coupling reaction:

   (FSO₂-)ₐCF₃₋ₐ(-COX^{a}) (1-1),

   R^{a}-COY^{a} (2-1),

   [(FSO₂-)ₐCF₃₋ₐ-]R^{a} (3-1)

   wherein symbols are as follows:
   a: an integer of from 1 to 3;
   X^{a}: a hydroxyl group or a group represented by the formula -OM;
   Y^{a}: a hydroxyl group or a group represented by the formula -OM;
   R^{a}: a monovalent organic group; and
   M: an alkali metal atom.
(5) The production method according to the above (4),
   wherein R^{a} is selected from the group consisting of a polyfluoroalkyl group, a polyfluoro(partially chloroalkyl) group, a polyfluoro(etheric oxygen atom-containing alkyl) group, a polyfluoro(partially chloro(etheric oxygen atom-containing alkyl)) group, a polyfluoroalkenyl group and a polyfluoro(etheric oxygen atom-containing alkenyl) group, and is a C₁₋₁₀ monovalent organic group.
(6) The production method according to the above (4) or (5), wherein the compound represented by the formula (2-1) is used in an amount of from 0.01 to 1.0 mol per 1 mol of the compound represented by the formula (1-1).
(7) A method for producing a compound represented by the following formula (3-2), which comprises subjecting a compound represented by the following formula (1-1) and a compound represented by the following formula (2-2) to electrolytic coupling reaction:

   (FSO₂-)ₐCF₃-ₐ(-COX^{a}) (1-1),

   R^{b}(-COY^{a})₂ (2-2),

   [(FSO₂-)ₐCF₃₋ₐ-]₂R^{b} (3-2)

   wherein symbols are as follows:
   a: an integer of from 1 to 3;
   X^{a}: a hydroxyl group or a group represented by the formula -OM;
   Y^{a}: a hydroxyl group or a group represented by the formula -OM;
   R^{b}: a bivalent organic group; and
   M: an alkali metal atom.
(8) The production method according to the above (7),
   wherein R^{b} is selected from the group consisting of a polyfluoroalkylene group, a polyfluoro(partially chloroalkylene) group, a polyfluoro(etheric oxygen atom-containing alkylene) group and a polyfluoro(partially chloro(etheric oxygen atom-containing alkylene)) group, and is a C₁₋₁₀ bivalent organic group.
(9) The production method according to the above (7) or (8), wherein the compound represented by the formula (2-2) is used in an amount of from 0.005 to 0.5 mol per 1 mol of the compound represented by the formula (1-1).
(10) The production method according to any one of the above (1) to (9), wherein the electrolytic coupling reaction is carried out in the presence of a solvent essentially containing a fluorinated alcohol.

### EFFECTS OF THE INVENTION

According to the production method of the present invention, fluorinated compounds having -SO₂F with various structures can efficiently be produced.

### BEST MODE FOR CARRYING OUT THE INVENTION

In the present specification, the groups are as defined above unless otherwise specified. A compound represented by the formula (1) will sometimes be referred to as a compound 1. The same applies to compounds represented by other formulae. A group represented by the formula -OM will sometimes be referred to as -OM. The pressure is the gauge pressure unless otherwise specified.

In the present specification, photocoupling reaction and electrolytic coupling reaction will sometimes collectively be referred to as coupling reaction.

In the present specification, a polyfluoro group means a group with a content of fluorine atoms of from 30 mass% to 86 mass% based on the total mass of the group. Further, a perfluoro group means a group having all hydrogen atoms bonded to carbon atoms substituted by fluorine atoms and having no hydrogen atom bonded to a carbon atom.

In the present specification, an organic group may contain, as a hetero atom (an atom other than carbon atoms and hydrogen atoms), a nitrogen atom, an oxygen atom, a sulfur atom, a halogen atom (such as a fluorine atom, a chlorine atom, a bromine atom or an iodine atom) or the like.

In the present specification, the organic group is preferably a hydrocarbon group, an oxygen-containing hydrocarbon group, a halogenohydrocarbon group or a halogeno(oxygen atom-containing hydrocarbon) group, particularly preferably a fluorohydrocarbon group, a fluoro(oxygen atom-containing hydrocarbon) group, a fluoro(partially chloro(hydrocarbon)) group or a fluoro(partially chloro(oxygen atom-containing hydrocarbon)) group. The oxygen atom in the oxygen atom-containing hydrocarbon is preferably contained as an etheric oxygen atom in the carbon-carbon bond of the group or as an oxygen atom constituting a carbonyl structure or an ester structure in the carbon-carbon bond of the group.

The organic group may be either a saturated group or an unsaturated group, and may be either an aliphatic group or an aromatic group. The organic group is preferably an aliphatic group. The structure of the organic group may, for example, be a linear structure, a branched structure, a cyclic structure or a structure partially having a cyclic structure. The number of carbon atoms in the organic group is preferably from 1 to 20, particularly preferably from 1 to 10.

In the production method of the present invention, the following compound 1 and the following compound 2 are subjected to photocoupling reaction when X and Y are a fluorine atom or to electrolytic coupling reaction when X is a hydroxyl group or -OM and Y is a hydroxyl group or -OM, to obtain the following compound 3 (a, b, n, X, Y, Z, R and M are as defined above):

(FSO₂-)ₐ(Z-)₃₋ₐC(-COX) (1),

R(-COY)_{b} (2),

[(FSO₂-)ₐ(Z-)₃₋ₐC-]ₙR(-COY)_{b-n} (3)

X and Y are each independently preferably a hydroxyl group or -OM, and the coupling reaction is preferably electrolytic coupling reaction. When X and Y are -OM, the two -OM's may be the same or different, preferably the same.

Z is preferably a fluorine atom, a chlorine atom or a polyfluoro monovalent hydrocarbon group (preferably a C₁₋₃ perfluoroalkyl group), more preferably a fluorine atom or a trifluoromethyl group, particularly preferably a fluorine atom. Further, when "a" is 1, the two Z's in the compound 1 may be the same or different.

M is preferably a lithium atom, a sodium atom or a potassium atom, and from the viewpoint of the solubility in a reaction solvent, it is particularly preferably a sodium atom or a potassium atom.

"a" represents the number of the fluorosulfonyl group and is preferably 1.

"b" represents the number of the -COY group bonded to the organic group R in the compound 2 and represents the number corresponding to the valence of the organic group R. "b" is an integer of from 1 to 4, preferably 1 or 2, particularly preferably 1.

"n" represents the number of the group represented by the formula [(FSO₂-)ₐ(Z-)₃₋ₐC-] in the compound 3 formed by coupling of 1 mol of radicals derived from the compound 2 with n mol of radicals (radicals represented by the formula (FSO₂-)ₐ(Z-)₃₋ₐC·) derived from the compound 1, and is an integer of at most "b". "n" is an integer of from 1 to 4, preferably 1 or 2, particularly preferably 1.

"b" and "n" are preferably the same (that is, no -COY group is preferably present in the compound 3), and they are each preferably 1 or 2, particularly preferably 1.

R is a b-valent organic group, preferably a b-valent aliphatic organic group, more preferably a b-valent organic group selected from the group consisting of an aliphatic hydrocarbon group, an etheric oxygen atom-containing aliphatic hydrocarbon group, a halogenoaliphatic hydrocarbon group and a halogeno(etheric oxygen atom-containing aliphatic hydrocarbon) group. Among them, particularly preferred is a b-valent organic group selected from the group consisting of a fluoroaliphatic hydrocarbon group, a fluoro(partially chloroaliphatic hydrocarbon) group, a fluoro(etheric oxygen atom-containing aliphatic hydrocarbon) group and a fluoro(partially chloro(etheric oxygen atom-containing aliphatic hydrocarbon)) group. The b-valent organic group selected from the above is preferably a polyfluoro group, particularly preferably a perfluoro group. In such a case, the compatibility of the compound 1 with the compound 2 is high, whereby the coupling reaction of the compounds 1 and 2 will proceed with a high yield. When R is a b-valent organic group, the number of carbon atoms in R is preferably an integer of from 1 to 10, particularly preferably an integer of from 1 to 6.

When b is 1, R is a monovalent organic group, preferably a monovalent organic group selected from the group consisting of an alkyl group, an etheric oxygen atom-containing alkyl group, an alkenyl group, an etheric oxygen atom-containing alkenyl group, a halogenoalkyl group, a halogeno(etheric oxygen atom-containing alkyl) group and a halogeno(etheric oxygen atom-containing alkenyl) group. It is more preferably a monovalent organic group selected from the group consisting of a polyfluoroalkyl group, a polyfluoro(partially chloroalkyl) group, a polyfluoro(etheric oxygen atom-containing alkyl) group, a polyfluoro(partially chloro(etheric oxygen atom-containing alkyl)) group, a polyfluoroalkenyl group and a polyfluoro(etheric oxygen atom-containing alkenyl) group. Among them, it is particularly preferably a monovalent organic group selected from the group consisting of a perfluoroalkyl group, a perfluoro(partially chloroalkyl) group, a perfluoro(etheric oxygen atom-containing alkyl) group, a perfluoro(partially chloro(etheric oxygen atom-containing alkyl)) group, a perfluoroalkenyl group and a perfluoro(etheric oxygen atom-containing alkenyl) group. When R is a monovalent organic group, the number of carbon atoms in R is preferably from 1 to 10, particularly preferably from 1 to 6.

When b is 2, R is a bivalent organic group, preferably a bivalent organic group selected from the group consisting of an alkylene group, an etheric oxygen atom-containing alkylene group, a halogenoalkylene group and a halogeno(etheric oxygen atom-containing alkylene) group. It is more preferably a bivalent organic group selected from the group consisting of a polyfluoroalkylene group, a polyfluoro(partially chloroalkylene) group, a polyfluoro(etheric oxygen atom-containing alkylene) group and a polyfluoro(partially chloro(etheric oxygen atom-containing alkylene)) group. Among them, it is particularly preferably a bivalent organic group selected from the group consisting of a perfluoroalkylene group, a perfluoro(partially chloroalkylene) group, a perfluoro(etheric oxygen atom-containing alkylene) group and a perfluoro(partially chloro(etheric oxygen atom-containing alkylene)) group. When R is a bivalent organic group, the number of carbon atoms in R is preferably from 1 to 10, particularly preferably from 1 to 6.

As specific examples of the compound 1, the following compounds may be mentioned (X^{a} is as defined above). Among them, the following compound 1-1 is preferred, and the following compound 1-11 is particularly preferred:

(FSO₂-)ₐCF₃₋ₐ-COX^{a} (1-1),

(FSO₂-)CF₂-COX^{a} (1-11),

(FSO₂-)CF(CF₃)-COX^{a},

(FSO₂-)₂C(CF₃)-COX^{a},

(FSO₂-)C(CF₃)₂-COX^{a},

(FSO₂-)CFCl-COX^{a},

(FSO₂-)₂CCl-COX^{a},

(FSO₂-)CCl₂-COX^{a},

As specific examples of the compound 1-1, the following compounds may be mentioned:

FSO₂-CF₂COF,

FSO₂-CF₂COOH,

FSO₂-CF₂COONa,

(FSO₂-)₂CFCOF,

(FSO₂-)₂CFCOOH,

(FSO₂-)₂CFCOONa,

(FSO₂-)₃CCOF,

(FSO₂-)₃CCOOH,

(FSO₂-)₃CCOONa.

The compound 2 is preferably the following compound 2-1 or 2-2, provided that R^{a}, R^{b}, Y^{a} and Y^{b} are as defined above:

R^{a}-COY^{a} (2-1),

R^{b}(-COY^{b})₂ (2-2).

R^{a} is a monovalent organic group, and the preferred embodiment of R^{a} is the same as that of R when b is 1. R^{a} is a group selected from the group consisting of a polyfluoroalkyl group, a polyfluoro(partially chloroalkyl) group, a polyfluoro(etheric oxygen atom-containing alkyl)group, a polyfluoro(partially chloro(etheric oxygen atom-containing alkyl)) group, a polyfluoroalkenyl group and a polyfluoro(etheric oxygen atom-containing alkenyl) group and is preferably a C₁₋₁₀ monovalent organic group.

R^{b} is a bivalent organic group, and the preferred embodiment of R^{b} is the same as that of R when b is 2. R^{b} is a group selected from the group consisting of a polyfluoroalkylene group, a polyfluoro(partially chloroalkylene) group, a polyfluoro(etheric oxygen atom-containing alkylene) group and a polyfluoro(partially chloro(etheric oxygen atom-containing alkylene)) group and is preferably a C₁₋₁₀ bivalent organic group.

Y^{a} and Y^{b} are each independently a hydroxyl group or a group represented by the formula -OM, and the preferred embodiment of Y^{a} and Y^{b} is the same as that of Y^{b}.

As specific examples of the compound 2-1, the following compounds may be mentioned:

CF₂=CFO(CF₂)₃COOH,

CF₂=CFO(CF₂)₃COONa,

CF₂ClCFClO(CF₂)₃COOH,

CF₂ClCFClO(CF₂)₃COONa,

CF₂=CF(CF₂)₃COOH,

CF₂=CF(CF₂)₃COONa,

CF₂ClCFCl(CF₂)₃COOH,

CF₂ClCFCl(CF₂)₃COONa,

CF₂ClCFClCOOH,

CF₂ClCFClCOONa,

CF₃CF₂COOH,

CF₃CF₂COONa,

F(CF₂)₄CF(CF₃)COOH,

F(CF₂)₄CF(CF₃)COONa.

As specific examples of the compound 2-2, the following compounds may be mentioned:

HOCO (CF₂) ₂COOH,

NaOCO(CF₂)₂COONa,

HOCO(CF₂)₃COOH,

NaOCO(CF₂)₃COONa,

HOCO(CF₂)₄COOH,

NaOCO(CF₂)₄COONa.

In the production method of the present invention, photocoupling reaction is carried out when X in the compound 1 and Y in the compound 2 are a fluorine atom, and electrolytic coupling reaction is carried out when X in the compound 1 and Y in the compound 2 are each independently a hydroxyl group or -OM.

In the coupling reaction, it is considered that the compound 3 is formed by crosswise coupling reaction (sometimes called cross coupling reaction) of radicals represented by the following formula (r1) formed by decarboxylation of the compound 1 and radicals represented by the following formula (r2) formed by decarboxylation of the compound 2 (in the formulae, "(·)ₙ" means that an n number of radicals are formed in the group R):

(FSO₂-)ₐ(Z-)₃₋ₐC (r1),

R(·)ₙ(-COY)_{b-n} (r2).

Further, the reaction product may contain, in addition to the desired compound 3, by-products such as a compound formed by coupling reaction of radicals derived from the compound 1 (such as the following compound a) and a compound formed by coupling reaction of radicals derived from the compound 2:

[(FS0₂-)ₐ(Z-)₃-ₐC-]₂ (a)

In such a case, it is preferred to subject the reaction product to post-treatment thereby to obtain the compound 3 having a purity depending upon the purpose of use. The post-treatment method may, for example, be washing with water, extraction, chromatography or distillation. As the post-treatment method, one of these methods may be employed or two or more methods may be employed in combination.

In the coupling reaction, it is preferred to use the compound 2 in an amount of (0.01/b to 1.0/b) mol, particularly preferably (0.05/b to 0.5/b) mol per 1 mol of the compound 1.

For the photocoupling reaction, a known means of electrode reaction may be applicable. A light source to be used for light irradiation is preferably a low-pressure mercury lamp, a medium-pressure mercury lamp or a high-pressure mercury lamp. The reaction temperature is preferably from -50 to -100°C, particularly preferably from 0 to +50°C in view of the reaction efficiency. The photocoupling reaction may be conducted in the absence of a solvent or may be conducted in the presence of a solvent inert to the photoreaction (e.g. a perfluorocarbon or a perfluoroether). The reaction pressure may be any of atmospheric pressure, reduced pressure and elevated pressure and is preferably atmospheric pressure.

For the electrolytic coupling reaction, a known means is applicable. The electrode of an electrolytic apparatus used in the electrolytic coupling reaction is preferably an electrode with a high oxidation-reduction potential (preferably a platinum electrode). The electric current density in the electrolytic coupling reaction is preferably from 0.01 to 1.0 A/cm², and preferably from 0.02 to 0.5 A/cm² from the viewpoint of the control of heat generation and the reaction efficiency.

The electrolytic cell in the electrolytic apparatus is preferably an electrolytic cell made of glass or an electrolytic cell made of a resin (a fluororesin). Further, the electrolytic coupling reaction may be conducted employing the main body of the electrolytic cell as the anode. The reaction pressure may be any of atmospheric pressure, reduced pressure and elevated pressure, and is preferably atmospheric pressure. The reaction temperature is from -20°C to +100°C, preferably from -20°C to +60°C.

The electrolytic coupling reaction may be conducted in the absence of a solvent or may be conducted in the presence of a solvent, and is preferably conducted in the presence of a solvent. The solvent is preferably a polar solvent, particularly preferably a polar protic solvent (a fluorinated alcohol such as CF₃CF₂CH₂OH, CF₃CH(OH)CF₃ or CF₃CH₂OH; a hydrocarbon alcohol such as CH₃OH or CH₃CH₂OH; or water) or a polar aprotic solvent (a nitrile such as CH₃CN). The solvents may be used alone or as a mixture of at least two. The solvent is preferably a solvent essentially containing a fluorinated alcohol, particularly preferably a solvent essentially containing a fluorinated alcohol and water, with a view to improving the reaction yield. Further, the amount of the fluorinated alcohol in the solvent is preferably from 10 to 40 vol%.

The electrolytic coupling reaction may be carried out in a batch manner or may be carried out in a continuous manner by continuously supplying the compound 1 or the compound 2 to the electrolytic apparatus. The latter is preferred in view of a high efficiency.

In the electrolytic coupling reaction, one or two types of each of the compounds 1 and 2 may be used. For example, a compound 1 in which X is a hydroxyl group and a compound 1 in which X is -OM may be used in combination. Further, a compound 2 in which Y is a hydroxyl group and a compound 2 in which Y is -OM may be used in combination. In a case where the electrolytic coupling reaction is conducted in the presence of water, it is preferred that a compound 1 in which X is a hydroxyl group and a compound 1 in which X is -OM are used in combination and that a compound 2 in which Y is a hydroxyl group and a compound 2 in which Y is -OM are used in combination.

When compounds 1 in which X is a hydroxyl group and -OM are used in combination, the respective compounds 1 may be mixed, or a compound in which X is a hydroxyl group is mixed with an alkaline compound (such as an alkali metal hydroxide) in the presence of water. The same applies to a method for preparing compounds 2 in which Y is a hydroxyl group and -OM.

In a case where the electrolytic coupling reaction is conducted by using compounds 1 in which X is a hydroxyl group and -OM and compounds 2 in which Y is a hydroxyl group and -OM, the amount ratio is such that a compound 1 and a compound 2 in which X and Y are -OM is preferably from 0.01 to 1.00 molar amount, particularly preferably from 0.01 to 0.10 molar amount, the total amount of a compound 1 and a compound 2 in which X and Y are a hydroxyl group, from the viewpoint of the reaction efficiency.

In the production method of the present invention, the compound 3 is preferably the following compound 3-1 obtained by subjecting the compounds 1-1 and 2-1 to electrolytic coupling reaction or the following compound 3-2 obtained by subjecting the compounds 1-1 and 2-2 to electrolytic coupling reaction:

[(FSO₂-)ₐCF₃₋ₐ-]R^{a} (3-1)

[(FSO₂-)ₐCF₃₋ₐ-]₂R^{b} (3-2)

As specific examples of the compound 3-1, the following compounds may be mentioned:

FSO₂CF₂-(CF₂)₃OCF=CF₂,

FSO₂CF₂-(CF₂)₃OCFClCF₂Cl,

FSO₂CF₂-(CF₂)₂CF=CF₂,

FSO₂CF₂-(CF₂) ₂CFClCF₂Cl,

FSO₂CF₂-CFClCF₂Cl,

FSO₂CF₂-CF₂CF₃,

FSO₂CF₂-CF₂(CF₃)O(CF₂)₄F.

As specific examples of the compound 3-2, the following compounds may be mentioned:

FSO₂CF₂-(CF₂)₂-CF₂SO₂F,

FSO₂CF₂-(CF₂)₃-CF₂SO₂F,

FSO₂CF₂-(CF₂)₄-CF₂SO₂F.

In the production method of the present invention, formation of the compound 3 by cross coupling reaction of the compound 1 without being decomposed with another carboxylic acid analogue (compound 2) is an unexpected effect. According to the production method of the present invention, fluorinated compounds (compound 3) having -SO₂F with various structures can be produced in a short step from readily available compounds.

### EXAMPLES

Now, the present invention will be described in further detail with reference to Examples. However, the present invention is not limited thereto. Hereinafter, FSO₂CF₂COOH is represented by compound 11, F(CF₂)₄OCF(CF₃)COOH by compound 21, and the desired product F(CF₂)₄OCF(CF₃)CF₂O₂F by compound 31.

### EXAMPLE 1: Example (1) for preparation of compound 31 in a batch manner

In a vial (made of glass, internal capacity 50 mL) in which a stirrer was put, 20.0 mL of water, 3.0 mL of CH₃CN, 5 mmoL of NaOH, 60.0 mmoL of compound 11 and 5.0 mmoL of compound 21 were charged under cooling with ice. Nets (made of platinum, 80 mesh, width 30 mm, height 30 mm) put in mesh bags (made of polyethylene) were placed in parallel with each other with a distance of 3 mm as an anode and a cathode in the vial. As the reference electrode, a silver/silver chloride electrode was used. The areas of the anode and the cathode immersed in the solution were respectively 9 cm².

An electric current of 1.0 A was applied between the electrodes (total amount of electric current applied: 5.0 mF) while stirring the content in the vial to obtain a liquid separated into two layers. Immediately after the application of an electric current started, vigorous bubbling was observed near the electrodes, and the voltage during the electric current application was from 3.6 to 4.2 V.

The lower layer in the liquid separated into two layers was recovered and analyzed by ¹⁹F-NMR (internal standard: hexafluorobenzene, solvent: CDCl₃) and as a result, formation of 0.62 mmoL of compound 31 was confirmed. The reaction yield from compound 21 was 12%.

Further, presence of 0.40 mmoL of FSO₂CF₂CF₂SO₂F, 0.32 mmoL of (F(CF₂)₄OCF(CF₃))₂ and 3.09 mmoL of compound 21 was confirmed.

The upper layer in the liquid separated into two layers was recovered and analyzed by ¹⁹F-NMR (internal standard: CF₃CH₂OH, solvent: D₂O) and as a result, presence of 51.0 mmoL of compound 11 and 0.25 mmoL of compound 21 was confirmed.

### EXAMPLE 2: Example (2) for preparation of compound 31 in a batch manner

In a vial (made of glass, internal capacity 50 mL) in which a stirrer was put, 20.0 mL of water, 3.0 mL of CH₃CN, 5 mmoL of Na₂CO₃, 10.0 mL of CF₃CF₂CH₂OH, 59.9 mmoL of compound 11 and 5.0 mmoL of compound 21 were charged under cooling with ice. Nets (made of platinum, 80 mesh, width 30 mm, height 30 mm) put in mesh bags (made of polyethylene) were placed in parallel with each other with a distance of 3 mm as an anode and a cathode in the vial. As the reference electrode, a silver/silver chloride electrode was used. The areas of the anode and the cathode immersed in the solution were respectively 9 cm².

An electric current of 1.0 A was applied between the electrodes (total amount of electric current applied: 41.8 mF) while stirring the content in the vial to obtain a liquid separated into two layers. Immediately after the application of an electric current started, vigorous bubbling was observed near the electrodes, and the voltage during the electric current application was from 4.2 to 5.5 V.

The lower layer in the liquid separated into two layers was recovered and analyzed by ¹⁹F-NMR (internal standard: hexafluorobenzene, solvent: CDCl₃) and as a result, formation of 2.36 mmoL of compound 31 was confirmed. The reaction yield from compound 21 was 47%.

Further, presence of 7.91 mmoL of FSO₂CF₂CF₂SO₂F, 0.19 mmoL of (F(CF₂)₄OCF(CF₃))₂ and 0.35 mmoL of compound 21 was confirmed.

The upper layer in the liquid separated into two layers was recovered and analyzed by ¹⁹F-NMR (internal standard: CF₃CH₂OH, solvent: D₂O) and as a result, presence of 23.5 mmoL of compound 11 and 0.37 mmoL of compound 21 was confirmed.

### EXAMPLE 3: Example for preparation of compound 31 in a continuous manner

In a vial (made of glass, internal capacity: 50 mL) in which a stirrer was put, 20.0 mL of water, 3.0 mL of CH₃CN, 5 mmoL of NaOH, 10.0 mL of CF₃CF₂CH₂OH, 59.9 mmoL of compound 11 and 5.0 mmoL of compound 21 were charged under cooling with ice. Nets (made of platinum, 80 mesh, width 30 mm, height 30 mm) put in mesh bags (made of polyethylene) were placed in parallel with each other with a distance of 3 mm as an anode and a cathode in the vial. As the reference electrode, a silver/silver chloride electrode was used. The areas of the anode and the cathode immersed in the solution were respectively 9 cm².

Application of an electric current of 1.0 A between electrodes was started while stirring the content in the vial. 6 mmoL of compound 11 and 1.0 mmoL of compound 21 were additionally put in the vial every electric current application amount of 5 mF until the total amount of electric current application became 60 mF. The total amount of compound 11 charged was 132.9 mmol and the total amount of compound 21 charged was 17.0 mmol after the electric current application started. Further, an electric current of 1.0 A was applied between the electrodes until the total amount of electric current application became 100 mF. The voltage during the electric current application was from 4.2 to 5.5 V.

The heavy layers in the vial when the amounts of electric current application were 15 mF, 30 mF, 45 mF, 60 mF, 80 mF and 100 mF were mixed, and the resulting reaction liquid was analyzed by ¹⁹F-NMR (internal standard: hexafluorobenzene, solvent: CDCl₃) and as a result, formation of 6.95 mmoL of compound 31 was confirmed. The reaction yield from compound 21 was 41%.

Further, formation of (F(CF₂)₄OCF(CF₃))₂ (27.7 mmoL) and FSO₂CF₂CF₂SO₂F (0.52 mmoL) was confirmed. Further, presence of 6.26 mmoL of compound 21 and 7.71 mmoL of compound 11 was confirmed. Further, the upper layer liquid in the vial after completion of the electric current application was analyzed by ¹⁹F-NMR (internal standard: CF₃CH₂OH, solvent: D₂O) and as a result, presence of 31.2 mmoL of compound 11 and 0.36 mmoL of compound 21 was confirmed.

### EXAMPLE 4: Example (1) for preparation of another compound 3

The desired product CF₃CF₂CF₂SO₂F is obtained by electrolytic coupling reaction in the same manner as in Example 2 except that 5 mmol of CF₃CF₂COOH is used instead of compound 21 in Example 2.

### EXAMPLE 5: Example (2) for preparation of another compound 3

The desired product CF₂ClCFClCF₂SO₂F is obtained by electrolytic coupling reaction in the same manner as in Example 2 except that 5 mmol of CF₂ClCFClCOOH is used instead of compound 21 in Example 2.

### EXAMPLE 6: Example (3) for preparation of another compound 3

The desired product FSO₂CF₂(CF₂)₃CF₂SO₂F is obtained by electrolytic coupling reaction in the same manner as in Example 2 except that 2.5 mmol of HOOC(CF₂)₃COOH is used instead of compound 21 in Example 2.

### INDUSTRIAL APPLICABILITY

According to the production method of the present invention, fluorinated compounds having -SO₂F with various structures can be produced in a short step from readily available compounds.

The entire disclosure of Japanese Patent Application No. 2005-119721 filed on April 18, 2005 including specification, claims, drawings and summary is incorporated herein by reference in its entirety.

## Claims

1. A method for producing a compound represented by the following formula (3), which comprises subjecting a compound represented by the following formula (1) and a compound represented by the following formula (2) to photocoupling reaction when X and Y are a fluorine atom, or to electrolytic coupling reaction when X and Y are each independently a hydroxyl group or a group represented by the formula -OM:
(FSO₂-)ₐ(Z-)₃₋ₐC(-COX) (1),
R(-COY)_{b} (2),
[(FSO₂)ₐ(Z-)₃₋ₐC-]ₙR(-COY)_{b-n} (3)
wherein symbols are as follows:
X: a fluorine atom, a hydroxyl group or a group represented by the formula -OM;
Y: a fluorine atom, a hydroxyl group or a group represented by the formula -OM;
Z: a hydrogen atom, a fluorine atom, a chlorine atom or a monovalent organic group;
R: a b-valent organic group;
M: an alkali metal atom;
a: an integer of from 1 to 3, and
b and n: each independently an integer of from 1 to 4, provided that b≧n.

2. The production method according to Claim 1, wherein R is a group selected from the group consisting of a fluoroaliphatic hydrocarbon group, a fluoro(partially chloroaliphatic hydrocarbon) group, a fluoro(etheric oxygen atom-containing aliphatic hydrocarbon) group and a fluoro(partially chloro(etheric oxygen atom-containing aliphatic hydrocarbon)) group, and is a C₁₋₁₀ b-valent organic group.

3. The production method according to Claim 1 or 2, wherein the compound represented by the formula (2) is used in an amount of from (0.01/b to 1.0/b) mol (wherein b is as defined above) per 1 mol of the compound represented by the formula (1).

4. A method for producing a compound represented by the following formula (3-1), which comprises subjecting a compound represented by the following formula (1-1) and a compound represented by the following formula (2-1) to electrolytic coupling reaction:
(FSO₂-)ₐCF₃₋ₐ(-COX^{a}) (1-1),
R^{a}-COY^{a} (2-1),
[(FSO₂-)ₐCF₃₋ₐ-]R^{a} (3-1)
wherein symbols are as follows:
a: an integer of from 1 to 3;
X^{a}: a hydroxyl group or a group represented by the formula -OM;
Y^{a}: a hydroxyl group or a group represented by the formula -OM;
R^{a}: a monovalent organic group; and
M: an alkali metal atom.

5. The production method according to Claim 4, wherein R^{a} is selected from the group consisting of a polyfluoroalkyl group, a polyfluoro(partially chloroalkyl) group, a polyfluoro(etheric oxygen atom-containing alkyl) group, a polyfluoro(partially chloro(etheric oxygen atom-containing alkyl)) group, a polyfluoroalkenyl group and a polyfluoro(etheric oxygen atom-containing alkenyl) group, and is a C₁₋₁₀ monovalent organic group.

6. The production method according to Claim 4 or 5, wherein the compound represented by the formula (2-1) is used in an amount of from 0.01 to 1.0 mol per 1 mol of the compound represented by the formula (1-1).

7. A method for producing a compound represented by the following formula (3-2), which comprises subjecting a compound represented by the following formula (1-1) and a compound represented by the following formula (2-2) to electrolytic coupling reaction:
(FSO₂-)ₐCF₃₋ₐ(-COX^{a}) (1-1),
R^{b}(-COY^{a})₂ (2-2),
[(FSO₂-)ₐCF₃₋ₐ-]₂R^{b} (3-2)
wherein symbols are as follows:
a: an integer of from 1 to 3;
X^{a}: a hydroxyl group or a group represented by the formula -OM;
Y^{a}: a hydroxyl group or a group represented by the formula -OM;
R^{b}: a bivalent organic group; and
M: an alkali metal atom.

8. The production method according to Claim 7, wherein R^{b} is selected from the group consisting of a polyfluoroalkylene group, a polyfluoro(partially chloroalkylene) group, a polyfluoro(etheric oxygen atom-containing alkylene) group and a polyfluoro(partially chloro(etheric oxygen atom-containing alkylene)) group, and is a C₁₋₁₀ bivalent organic group.

9. The production method according to Claim 7 or 8, wherein the compound represented by the formula (2-2) is used in an amount of from 0.005 to 0.5 mol per 1 mol of the compound represented by the formula (1-1).

10. The production method according to any one of Claims 1 to 9, wherein the electrolytic coupling reaction is carried out in the presence of a solvent essentially containing a fluorinated alcohol.
